# EUROPEAN PATENT APPLICATION

(11) **EP 3 909 538 A1**
(43) Date of publication of application: **17.11.2021**
(21) Application number: 20174700.3
(22) Date of filing: 14.05.2020
(51) Int. Cl.: A61B 34/30, A61B 90/00, A61B 10/00, A61B 10/02

(54) **A SAMPLE OBTAINING DEVICE**

(71) Applicant: Syddansk Universitet, 5230 Odense M (DK)
(72) Inventor: Savarimuthu, Thiusius, Rajeeth, 5250 Odense SV (DK); Petersen, Henrik, Gordon, 5210 Odense SV (DK); Iversen, Nicolai, 5000 Odense C (DK); Kramberger, Aljaz, 5220 Odense SØ (DK); San Juan, Iñigo, Iturrate, 5000 Odense C (DK); Wilm, Jakob, 5000 Odense C (DK); Schmidt, Michael, Kjær, 5240 Odense NØ (DK); Denisa, Miha, 5230 Odense M (DK); Lindvig, Anders, Prier, 5000 Odense C (DK); Buch, Anders, Glent, 5240 Odense NØ (DK); Winther, Trine, Straarup, 5000 Odense C (DK); Sloth, Christoffer, 5000 Odense C (DK)
(74) Representative: Plougmann Vingtoft a/s

(57) **Abstract**

A device for obtaining a sample from a subject. The device has a base part (10) with a base canal (13) extending into the base part (10), sensor means (12) being adapted to detect an element entering the base canal, a holding part (100) with a coupling end configured to interlock with the base part (10), a receiving end, and a holding canal (101) for holding an associated sample obtaining element (5). The device is arranged for holding an associated sample obtaining element (5), having a sample end (6) which protrudes from the receiving end of the holding part (100) when the associated sample obtaining element is inserted into the holding canal. The base part has fixation means configured to provide releasable fixation in an axial direction, to the associated sample obtaining element, the fixation means fixating the associated sample obtaining element with a force F. The fixation means has safety means configured to release fixation of the associated sample obtaining element (5), if a force Q exceeding F, is applied to the sample end of the associated sample obtaining element, and the device is adapted to provide a signal to the associated device if the safety means or the sensor means are activated by the associated sample obtaining element (5), due to an external force, provided to the sample end (6) of the associated sample element in a longitudinal or substantially longitudinal direction.

## Description

### FIELD OF THE INVENTION

The present invention relates to a device and method for obtaining a sample from a patient.

### BACKGROUND OF THE INVENTION

The spread of virus through the global populous is recurrent in history, the most resent being the coronavirus which emerged in the Asian continent in late 2019 where it was identified in early 2020 and was later named COVID-19. The virus spread rapidly to other areas of the word in the following period. The COVID-19 outbreak was soon thereafter declared a pandemic.

Previously in history, the world has witnessed other virus based pandemics, such as the "Spanish flu" pandemic of 1918, the avian Influenza A H5N1 virus ("Bird flu") epidemic of 2004, and the H 1N 1 viral strain ("Swine flu") pandemic of 2009. These pandemics cause global problems, both economically and medically. Besides these virus-based pandemics, influenza virus epidemics are frequently reoccurring.

In order to determine if a human being is infected with a virus, such as for instance the COVID-19, health-care staff perform a throat swab for collecting a sample swab from the throat. The aim of the swabbing procedure is to collect a high number of virus particles from the patient's throat. There is evidence that sampling should occur from the posterior parts of the oral cavity and the posterior nasopharyngeal wall of the mouth of the patient.

The inhalation of air contaminated by harmful virus and/or other microorganisms is a common route for infection of human beings, particularly health-care staff and others working with infected humans or animals. Air exhaled by infected patients is a source of contamination. Masks are used as a barrier to prevent species-to-species transmission of the virus. However, especially health-care staff taking throat samples from patients are particularly exposed, and although they are wearing masks and similar barriers, there is a risk of cross-contamination between health-care staff and patients in the sample obtaining operation.

In order to control a pandemic outbreak a vast amount of swab tests must be taken. This increases this contamination risk but is reduced by using protective clothes, masks, protective eyewear and the like. This however, is costly as all of these protective measures are for single use or must be sterilised between each use.

Hence, an improved device and method for obtaining samples from patients would be advantageous.

### OBJECT OF THE INVENTION

In order to reduce the risk of cross contamination the idea has arisen of creating a device and method suitable for mounting on an automated system, for collecting a sample swab from the throat. Accordingly, an objective for the present invention is to provide a device enabling automated throat swabs on humans.

### SUMMARY OF THE INVENTION

Thus, the above-described object and several other objects are intended to be obtained in a first aspect of the invention by providing a device for obtaining a sample from a subject, said device comprising:
- a base part comprising a base canal extending into the base part,
- sensor means being adapted to detect an element entering the base canal,
- output means for outputting a signal from the sensor means to an associated device,
- a holding part, the holding part comprising a coupling end configured to interlocking with the base part, a receiving end and, a holding canal for holding a sample obtaining element, wherein the base canal and the holding canal are arrange coaxially when the base part and the holding part are interlocked,
- an associated sample obtaining element, having a sample end and a second end opposite to the sample end and wherein the sample end protrudes from the receiving end of the holding part when the associated sample obtaining element is inserted into the holding canal,
- fixation means configured to provide releasable fixation in an axial direction, to the associated sample obtaining element, the fixation means fixating the associated sample obtaining element with a force F, the fixation means comprising safety means configured to release fixation of the associated sample obtaining element, if a force Q exceeding F, is applied to the sample end of the associated sample obtaining element, and
wherein the device is adapted to provide a signal to the associated device if the safety means or the sensor means are activated by the associated sample obtaining element, due to an external force, provided to the sample end of the associated sample element in a longitudinal or substantially longitudinal direction.

In the context of the present invention, a canal is to be understood as a lumen or a channel, which can be accessed from at least one surface of the base part. The canal may be a through-hole or a bore, and the diametrical geometry of the canal may be a square, triangular, circular, hexagon or other suitable shape. In an embodiment of the invention, the shape of the canal may change, in an axial direction, during the path of the canal.

In the context of the present invention sensor means is to be understood as a device suitable for detecting an element or an obstacle entering the canal of the base part, such a magnetic sensor, a light sensor, an electro-mechanical switching element, a pressure sensor or other suitable sensor means.

In the context of the present invention, output means is to be understood as a mechanical or electrical interface to a secondary mechanical or electrical interface, such as a Wi-Fi or Bluetooth transmitter or a wired output device, such as a data transmission cable between the output means and a computer or other data processing means.

In the context of the present invention, coupling is to be understood as a device that serves to connect the ends of adjacent parts or objects, such as, but not limited to, a threaded coupling, a snap coupling, a muff or sleeve coupling, a magnetic coupling or a grip coupling.

In the context of the present invention, interlocking is to be understood as to connect two or more parts, so that the motion or operation of any part is constrained by another, i.e. the holding part is constrained from movements relative to the base part, when said two parts are interlocked.

In the context of the present invention, coaxially is to be understood as bringing the longitudinal axis's of the base canal and the holding canal together so as to form one elongate canal from said two canals.

In the context of the present invention, an associated sample obtaining element may be understood as, but not limited to e.g. a cotton swab, a sample tissue, pliers or other suitable sample obtaining element for obtaining a sample, such as a tissue sample, from a subject's mouth, throat, nasal cavity, ear cavity, skin or a hair sample.
In another embodiment of the invention, the sample may be an intra-cavitary sample, such as a rectal, vaginal or urethral swab.

In the context of the present invention, fixation means may be understood as mechanical gripping means, a magnetic field, a chemical adhesion or chemical friction element, a physical releasable barrier positioned in the canal, or other suitable means for releasable fixation of the associated sample obtaining element, so as to enable said sample obtaining element to move freely in an axial direction, relative to the canal. In an embodiment of the invention, the fixation means may provide flexible fixation, so as to allow the associated sample obtaining element to translate in an axial distance, such as no more than 1 mm, no more than 2 mm, no more than 3 mm, no more than 4 mm, no more than 5 mm or up to 100 mm.

In the context of the present invention, safety means may be understood as detection means, such as a light sensor, a pressure sensor, a magnetic sensor or a switch suitable for being activated if a pressure exceeding F is applied to the associated sample obtaining element and wherein the safety means enables said sample obtaining element to move freely in an axial direction, relative to the canal.

In a preferred embodiment of the invention, the base part is configured to be mounted on an automatic positioning device, and wherein the output means are connected to a controller or data processing means of the automatic positioning device, so as to send a stop signal and/or a signal for correcting the movement of the automatic positioning means.

In the context of the present invention, automatic positioning means may be understood as a robotic arm, a multidirectional automatic movement apparatus, a two-directional automatic movement apparatus or such as a rail-guided system in which the base part may be attached to said rail-guided system.

In an embodiment of the invention, receiving end of the holding part is positioned opposite, or substantially opposite to the coupling end.

In another preferred embodiment of the invention, the fixation means further comprise a resistive axial sliding mechanism, such as a spring or magnetic mechanism for enabling an axial movement of the associated sample obtaining element, wherein said resistive axial sliding mechanism would enable the associated sample obtaining element to move, in a controlled manner, axially within the holding canal, without triggering the safety means, before exceeding the force F or before triggering the measuring means. In an embodiment of the invention, the resistive axial sliding mechanism may enable the associated sample obtaining element to move such as 1 mm, such as 2 mm, such as 3 mm, such as 4 mm, such as 5 mm, such as 6 mm, such as 7 mm, such as 8 mm, such as 9 mm, such as 10 mm or such as up to 50 mm, in an axial direction, before triggering the measuring means or exceeding the force F, so as to activate the safety means.

In another embodiment of the invention, the base part further comprise distance measuring means for measuring a distance D and wherein the distance D is an axial movement between a start position and an end position of the associated sample obtaining element, relative to the holding part and/or the base part, during a sample obtaining process. This embodiment is particularly advantageous for outputting information regarding the current position of said sample obtaining element, relative to the holding canal, during a sample obtaining process. It is to be understood, that the distance data may be used to calculate a current force exerted to the sample obtaining element, based on a current positon of said sample obtaining element within the holding canal, relative to the start position of said sample obtaining element.

In an advantageous embodiment of the invention, the force F may be a force of no more than 100 newton, preferably a force of no more than 10 newton, more preferably a force of no more than 3 newton, even more preferably a force of no more than 1 newton, even more preferably a force of no more than 0.8 newton or most preferably a force of no more than 0.7 newton.

In another embodiment of the invention, the safety means are configured to measure axial and/or transverse forces applied to the fixation means from the associated sample obtaining element. It is to be understood that transverse or axial forces applied to the fixation means are transferred from an external transverse or axial force applied to the sample end of the associated sample obtaining element.

In a preferred embodiment of the invention, the holding part may be manufactured from a recyclable material, such as a suitable recyclable polymer, such as PETE, HDPE, LDPE, PVC or POM.

In another embodiment, the holding part may be manufactured from a disposable material, such as PMMA, SLA, SLS or PTFE.

In yet another embodiment of the invention, the holding part may be manufactured from a metal, such as stainless steel or other suitable metals.

In another preferred embodiment of the invention, the holding part is manufactured from a biodegradable material, such as a biodegradable polymer, such as polyglycolide, polylactide or polyhydroxobutyrate.

In an advantageous embodiment of the invention, the base part may be manufactured from a biocidal or antimicrobial material, such as a polymer synthesized with an antimicrobial agent.

In another advantageous embodiment, the base part may be manufactured from an antimicrobial metal, such as, but not limited to a copper alloy.

In a preferred embodiment of the invention, the base part may be coated with a biocidal agent or antimicrobial coating, such as, but not limited to, a parylene polymer.

In another embodiment of the invention, the base part may be manufactured from a metal, such as stainless steel or a suitable alloy for medical use.

In another preferred embodiment of the invention, the sample obtained by the associated sample obtaining element is a mouth/throat swab so as to sample tissue from a subject for further analysis, such as for pathogenic analysis, such as a mouth swab to test for Covid-19, tuberculosis or another pathogen, bacteria or virus.

In an embodiment of the invention, the safety means is a barrier or membrane, said barrier being penetrable by the force F applied by the second end of the associated sample obtaining element. It is to be understood that the safety means may function as a mechanical end stop to the second end of the associated sample obtaining element, and wherein the safety means, if penetrated by the sample obtaining element, enables the sample obtaining element to move freely in an axial direction, and, if moving past a threshold, triggering the measuring means and sending a signal to the associated device, such as sending a stop signal to a robotic arm to prevent any harm to a subject during a sample obtaining process.

In an embodiment of the invention the safety means is an electronic sensor, such as, but not limited to, a strain gauge, the electronic sensor being configured to detect a force applied to the sample end and/or from the second end of the associated sample obtaining device in a longitudinal or substantially longitudinal direction and wherein said force may be measured by the safety means or indirectly through the fixation means.

In another embodiment the holding canal and/or the base canal are conical or taped, so as to enable the associated sample obtaining element to be free from an inside wall, towards the receiving end of the holding tool and said canals being more narrow towards the base part, opposite the holding part.

In a preferred embodiment of the invention, the fixation means and/or the safety means is a magnetic device wherein the magnetic device is configured to detect a force applied to the sample end and/or from the second end of the associated sample obtaining device in a longitudinal or substantially longitudinal direction and upon detection of a force greater than F, release the fixation means and release an axial translation of the associated sample obtaining device so as to trigger a signal to the associated device and enable the sample end of the sample obtaining device to move in a direction opposite to the subject, from which, said sample is obtained. It is to be understood that this embodiment is advantageous for preventing any harm to said subject, by enabling the sample obtaining element to be free from fixation, and, upon applied pressure, move into the holding canal, away from said subject.

The invention is particularly, but not exclusively, advantageous for obtaining a sample of a subject, in which the subject is not exposed to physical harm stemming from excessive force applied to said subject, by an associated sample obtaining element mounted on the invention, and wherein the invention enable said sample obtaining element to be axially released and free to move into the holding part and/or base part of the device, if the force applied to said subject exceeds a threshold.

The device is further advantageous for being arranged on automatic positioning means, such as a robotic arm, in which the robotic arms force constraints are greater than the force constraints of the fixation means and safety means, thus enabling the device, and hence the automatic positioning means, to have an increased force sensitivity towards a subject, which are to be subject to forces applied to a sensitive area, such as the throat, without compromising the safety of said subject due to any force applied by the automatic positioning means.

The device is also further advantageous for obtaining a plurality of samples from a plurality of subjects, decreasing the risk of infecting a second subject with any particles from a first subject, due to the biocidal and/or antimicrobial properties of the holding part, or by discarding the holding part between any subsequent sample obtaining processes.

In a second aspect, the invention relates to a method for obtaining a sample from a subject, the method comprising:
- providing a base part comprising a base canal extending into the base part,
- providing sensor means being adapted to detect an element entering the base canal,
- providing output means for outputting a signal from the sensor means to an associated device,
- providing a holding part, the holding part comprising a coupling end configured to interlocking with the base part and a receiving end opposite to the coupling end, a holding canal for holding a sample obtaining element, wherein the base canal and the holding canal are arrange coaxially when the base part and the holding part are interlocked,
- providing fixation means configured to provide releasable fixation in an axial direction, to the associated sample obtaining element, the fixation means fixating the associated sample obtaining element with a force F, the fixation means comprising safety means configured to release fixation of the associated sample obtaining element, if a force Q exceeding F, is applied to the sample end of the associated sample obtaining element,
- providing the associated sample obtaining element, having a sample end and a second end opposite to the sample end and wherein the sample end protrudes from the receiving end of the holding part when the associated sample obtaining element is inserted into the holding canal,
- interlocking the holding part to the base part,
- inserting the associated sample obtaining element into the holding canal,
- obtaining a sample from the subject, such as from the subject's mouth or throat, such as a throat swab, and
wherein the method further comprises stopping the sample obtaining of the subject, if a force Q, which exceeds F, is applied to the sample end of the associated sample element in a longitudinal or substantially longitudinal direction and release fixation of the associated sample obtaining element.

In another embodiment of the invention, the associated sample obtaining element may already be inserted in the holding canal, when interlocking the holding part to the base part.

In an advantageous embodiment, the associated sample obtaining element and the holding part may be assembled and bundled together prior to use of the invention.

In a preferred embodiment of the invention, the method further comprises providing automatic positioning means, such as a robotic arm, wherein the positioning means is interlocked with the base part, and the associated sample obtaining element, or at least a part of said sample obtaining element, is inserted into an associated container, such as a sample vial after a sample has been obtained from a subject. As an example, a mouth swab from a subject, applied to a cotton swab is inserted into a sample vial, after which, at least the cotton swab and possibly the holding part are disposable.

In another preferred embodiment of the invention, the method further comprises moving the device according to the first aspect of the invention in at least a first direction transverse to a longitudinal axis of the associated container and breaking or snapping at least the sample end of the associated sample obtaining element into the associated sample container.

In another embodiment, the associated sample obtaining element may be inserted into a liquid within an associated container, so as to transfer at least some of the particles from the sample end of the associated sample obtaining element to said liquid and subsequently remove the sample obtaining element from the associated container.

In another embodiment, the associated sample obtaining element is inserted into an associated container, and while being inserted in said container, being disengaged from the holding part, leaving the sample obtaining element within said container.

In an advantageous embodiment of the invention, the method further comprises obtaining a plurality of samples from one or more subjects by including one or more of the following steps:
- discarding the holding part according the first aspect of the invention, from the base part when a sample has been deposited in the associated sample vial,
- initiating a disinfection process of the base part,
- initiating a second sample obtaining process according the second aspect of the invention,
- looping the method according to any of the steps, according to the second aspect of the invention, completing n sample obtaining processes.

In the context of the present invention, looping is to be understood as repeating one or more of the process steps according to the second aspect of the invention until an individual sample has been obtained, from all of the plurality of subjects.

This aspect of the invention is particularly, but not exclusively, advantageous in that the method according to the present invention may be implemented by using existing automated systems and implementing one or more of the parts according the first aspect of the invention so as to create a controlled process for automatic sample obtaining from one or more subjects, decreasing the risk of infection spreading.

In a third aspect, the invention relates to a kit for obtaining a sample from a subject, the kit comprising:
- a device according to the first aspect of the invention,
- a sample obtaining element comprising a sample end and a second end, the sample obtaining element being configured to break/snap at a breaking/snapping section of the sample obtaining element, if shear force exceeding between 0.1 and 20 newton, preferably between 0.5 and 10 newton or more preferably between 0.5 and 4 newton is applied to the sample obtaining element,
wherein the breaking/snapping section of the sample obtaining element is positioned at the receiving end of the holding part, when the sample obtaining element is inserted into the holding part, pressing against the safety means.

This aspect of the invention is particularly, but not exclusively, advantageous in that the kit according to the present invention may be implemented by using existing automated systems, such as a robot cell, and implementing one or more of the parts according the first aspect of the invention and the method according to the second aspect of the invention, so as to create a controlled process for automatic sample obtaining from one or more subjects, decreasing the risk of infection spreading.

In a fourth aspect, the invention relates to use of the device or kit according to the first and/or third aspect of the invention, for obtaining one or more samples from one or more subjects, using the method according to the second aspect of the invention.

The first, second, third and fourth aspect of the present invention may each be combined with any of the other aspects. These and other aspects of the invention will be apparent from and elucidated with reference to the embodiments described hereinafter.

### BRIEF DESCRIPTION OF THE FIGURES

The device for obtaining a sample from a subject, according to the invention, will now be described in more detail with regard to the accompanying figures. The figures show one way of implementing the present invention and is not to be construed as being limiting to other possible embodiments falling within the scope of the attached claim set.
Figure 1 is a trimetric view of a device, according to an embodiment of the invention.
Figure 2 is an exploded view of a device, according to an embodiment of the invention.
Figure 3 is a front view of a device, according to an embodiment of the invention.
Figure 4 is a side view of a device, according to an embodiment of the invention.
Figure 5 is a side view cross-section of a device, according to an embodiment of the invention.
Figure 6 is a side view of a device mounted on automatic positioning means, according to an embodiment of the invention.
Figure 7 is a flow-chart of a method according to the invention.
Figure 8 is a flow-chart of an additional method according to the invention.

### DETAILED DESCRIPTION OF AN EMBODIMENT

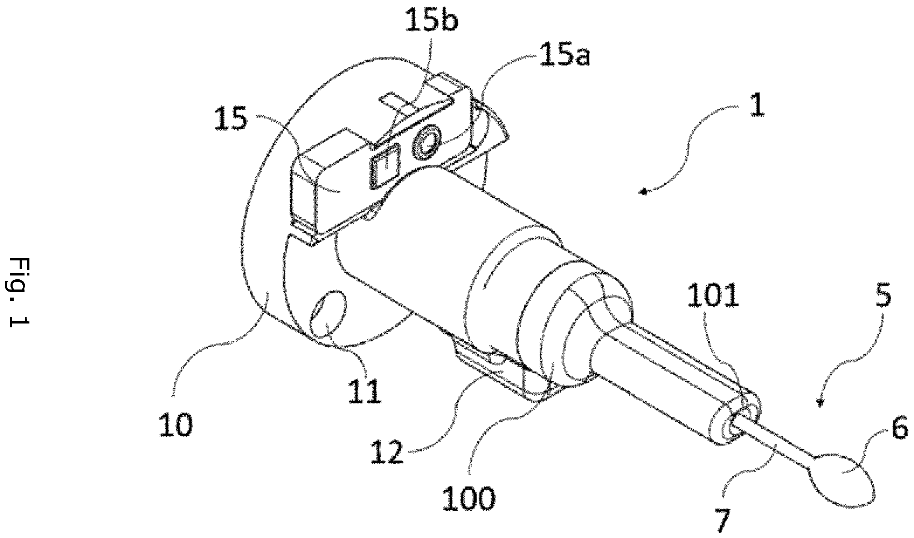

Figure 1 is a trimetric view of a device, according to an embodiment of the invention. Figure 1 illustrates an embodiment of an assembled sample tool 1, comprising a base part 10, a holding part 100, and an associated sample obtaining element 5, which is inserted into the holding part 100. On the base part 10, an image sensor device 15 is positioned, facing towards the associated sample obtaining element 5. The image sensor device 15 has a light emitting element 15a and a light sensor element 15b. The base part 10 further has a mounting hole 11, for mounting the base part to an associated device, such as a robotic arm (not shown) and sensor means 12 mounted on a bottom portion of the base part 10, towards the holding part 100. The holding part 100 is mounted to the base part 10, and the holding part 100 has a holding canal 101, in which a shaft 7 of the associated sample obtaining element 5 is inserted and wherein a sample end 6 of the associated sample element 5 protrudes from the sample tool 1.

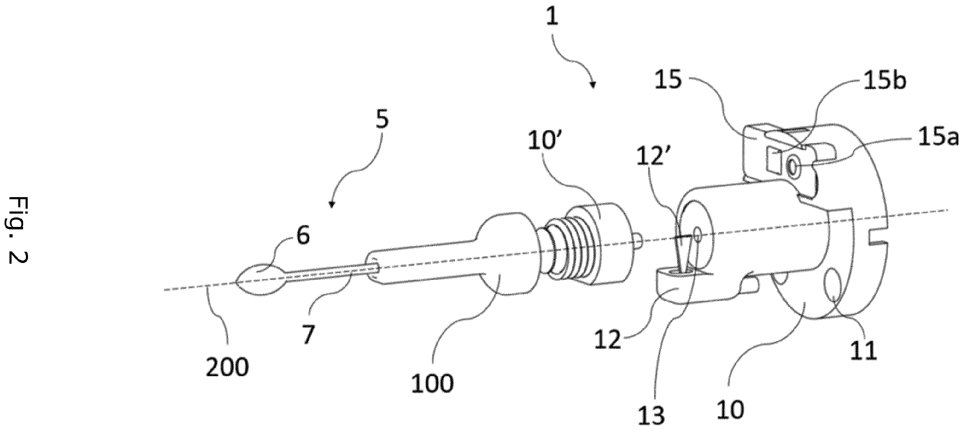

Figure 2 is an exploded view of a device, according to an embodiment of the invention. Figure 2 illustrates an embodiment of an assembled sample tool 1, in an exploded view comprising a first base part 10 and a second base part element 10' with a threaded end, a holding part 100, and an associated sample obtaining element 5, which is inserted into the holding part 100. On the base part 10, an image sensor device 15 is positioned, facing towards the associated sample obtaining element 5 and a base canal 13 centred in the base part 10, on a longitudinal axis 200. The image sensor device 15 has a light emitting element 15a and a light sensor element 15b. The base part 10 further has a mounting hole 11, for mounting the base part to an associated device, such as a robotic arm (not shown) and sensor means 12 mounted on a bottom portion of the base part 10, towards the holding part 100, with an illustration of light 12' emitted from the sensor means 12, towards the base canal 13. The holding part 100 is mounted to the base part 10, and the holding part 100 has a holding canal 101, in which a shaft 7 of the associated sample obtaining element 5 is inserted and wherein a sample end 6 of the associated sample element 5 protrudes from the sample tool 1.

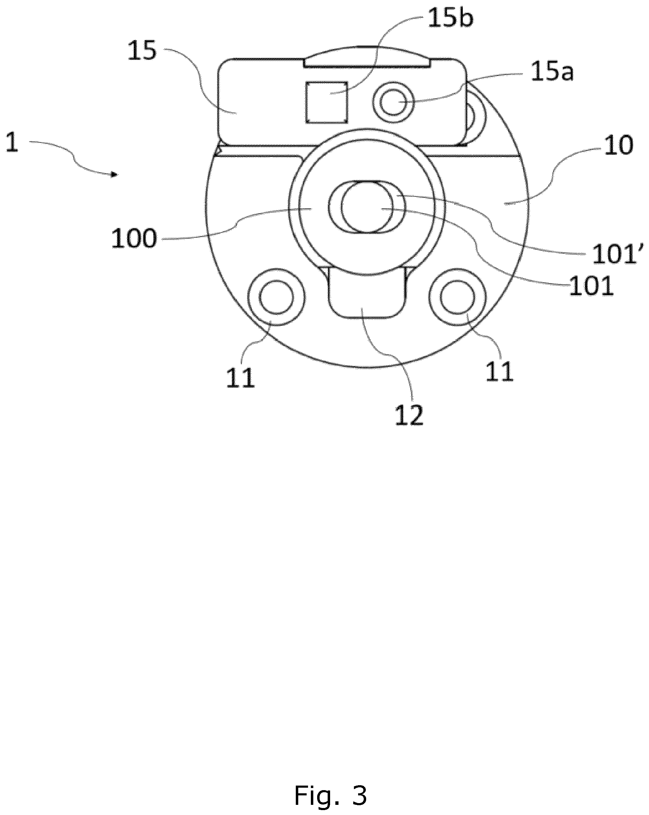

Figure 3 is a front view of a device, according to an embodiment of the invention. Figure 3 illustrates an embodiment of an assembled sample tool 1, comprising a first base part 10 and a holding part 100. On the base part 10, an image sensor device 15 is positioned. The image sensor device 15 has a light emitting element 15a and a light sensor element 15b. The base part 10 further has two mounting holes 11, for mounting the base part to an associated device, such as a robotic arm (not shown) and sensor means 12 mounted on a bottom portion of the base part 10, towards the holding part 100. The holding part 100 is mounted to the base part 10, and the holding part 100 has a holding canal 101 with as recess 101'.

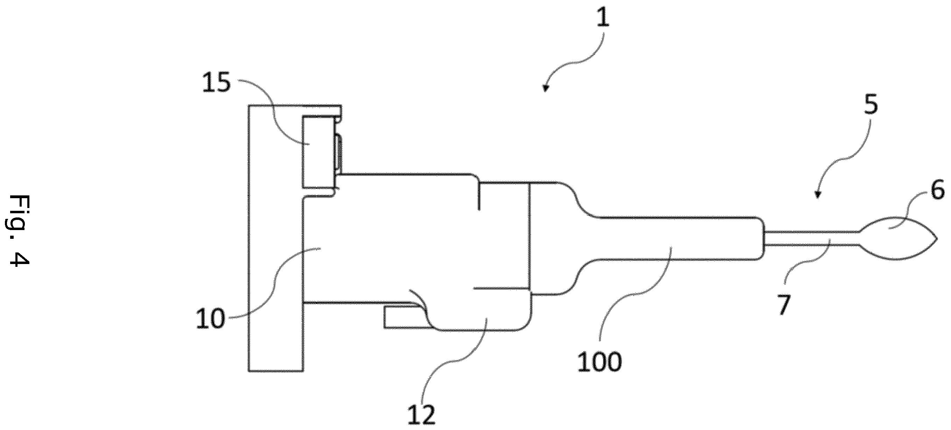

Figure 4 is a side view of a device, according to an embodiment of the invention. Figure 4 illustrates an embodiment of an assembled sample tool 1, comprising a first base part 10, a holding part 100 and an associated sample obtaining element 5, which is inserted into the holding part 100. On the base part 10, an image sensor device 15 is positioned. The base part 10 further has sensor means 12 mounted on a bottom portion of the base part 10, towards the holding part 100. The holding part 100 is mounted to the base part 10, and the holding part 100 in which a shaft 7 of the associated sample obtaining element 5 is inserted and wherein a sample end 6 of the associated sample element 5 protrudes from the sample tool 1.

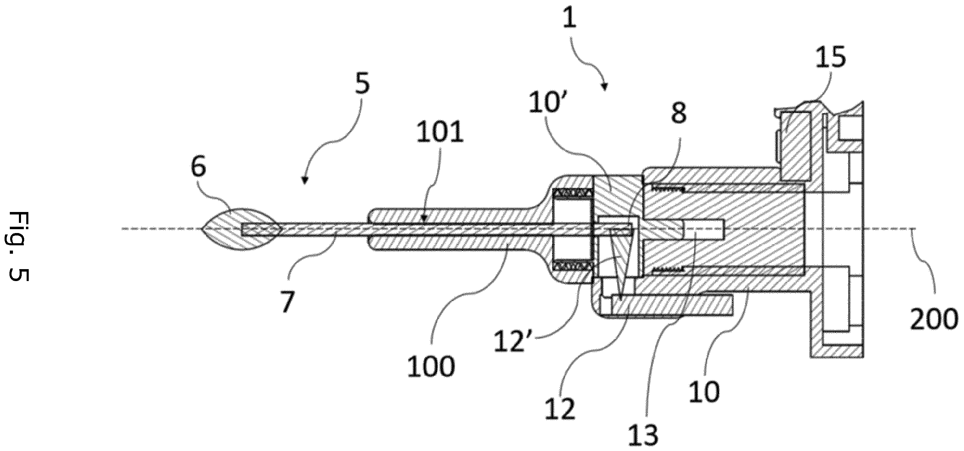

Figure 5 is a side view cross-section of a device, according to an embodiment of the invention. Figure 5 illustrates a cross-section of an assembled sample tool 1, comprising a first base part 10 and a second base part element 10' with a threaded end, a holding part 100, and an associated sample obtaining element 5, which is inserted into the holding part 100. On the base part 10, an image sensor device 15 is positioned, facing towards the associated sample obtaining element 5 and a base canal 13 centred in the base part 10, on a longitudinal axis 200. The base part 10 further sensor means 12 mounted on a bottom portion of the base part 10, towards the holding part 100, with an illustration of light 12' emitted from the sensor means 12, towards the base canal 13 and a second end 8 of the associated sample obtaining element 5. The holding part 100 is mounted to the base part 10, and the holding part 100 has a holding canal 101, in which a shaft 7 of the associated sample obtaining element 5 is inserted and wherein a sample end 6 of the associated sample element 5 protrudes from the sample tool 1.

The sample tool 1 is configured to send a signal to an associated device, such as automatic positioning means, such as a robotic arm, if the sensor means 12 detects the presence of the second end 8, of the associated sample obtaining element 5, within the base canal 13.

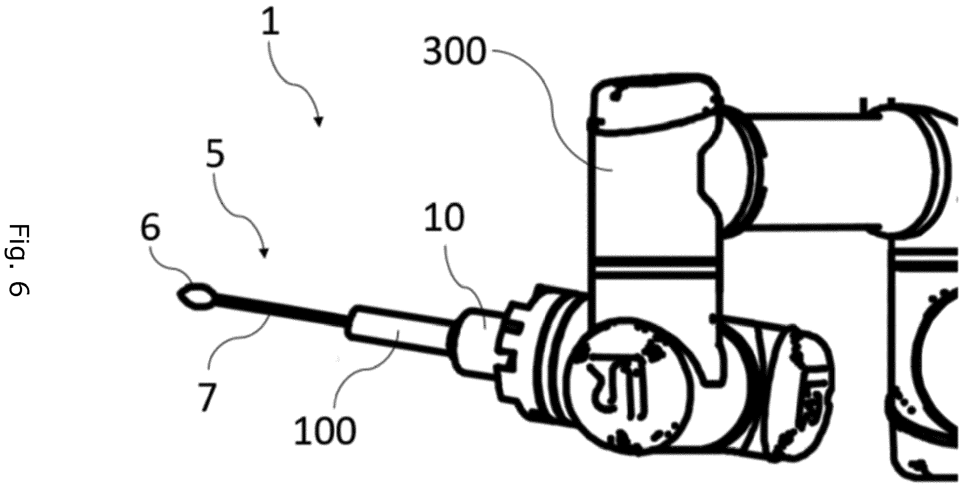

Figure 6 is a side view of a device mounted on automatic positioning means, according to an embodiment of the invention. Figure 6 illustrates an embodiment of an assembled sample tool 1 mounted on a robotic arm 300, the assembled sample tool comprising a first base part 10, a holding part 100 and an associated sample obtaining element 5, which is inserted into the holding part 100. The holding part 100 is mounted to the base part 10, and the holding part 100 in which a shaft 7 of the associated sample obtaining element 5 is inserted and wherein a sample end 6 of the associated sample element 5 protrudes from the sample tool 1.

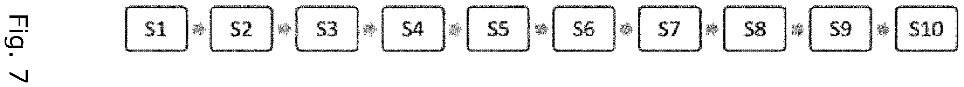

Figure 7 is a flow-chart of a method according to the invention. The flow-chart is a method embodiment for obtaining a sample from a subject, the method comprising:
S1 -providing a base part comprising a base canal extending into the base part,
S2 -providing sensor means being adapted to detect an element entering the base canal,
S3 -providing output means for outputting a signal from the sensor means to an associated device,
S4 -providing a holding part, the holding part comprising a coupling end configured to interlocking with the base part and a receiving end opposite to the coupling end, a holding canal for holding a sample obtaining element, wherein the base canal and the holding canal are arrange coaxially when the base part and the holding part are interlocked,
S5 -providing fixation means configured to provide releasable fixation in an axial direction, to the associated sample obtaining element, the fixation means fixating the associated sample obtaining element with a force F, the fixation means comprising safety means configured to release fixation of the associated sample obtaining element, if a force Q exceeding F, is applied to the sample end of the associated sample obtaining element,
S6 -providing the associated sample obtaining element, having a sample end and a second end opposite to the sample end and wherein the sample end protrudes from the receiving end of the holding part when the associated sample obtaining element is inserted into the holding canal and, by an external force,
S7 -interlocking the holding part to the base part,
S8 -inserting the associated sample obtaining element into the holding canal,
S9 -obtaining a sample from the subject, such as from the subjects mouth or throat, such as a throat swab, and
S10 -wherein the method further comprises stopping the sample obtaining of the subject, if a force Q, which exceeds F, is applied to the sample end of the associated sample element in a longitudinal or substantially longitudinal direction and release fixation of the associated sample obtaining element.

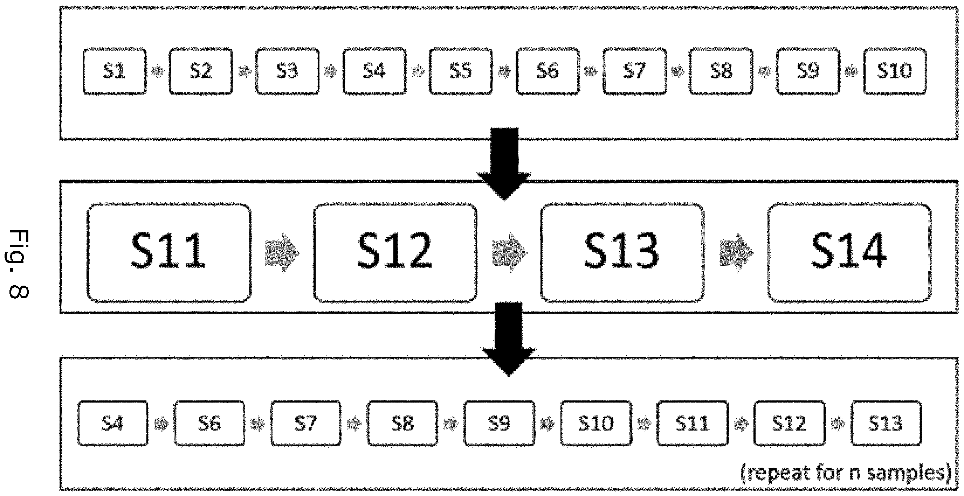

Figure 8 is a flow-chart of a second method according to the invention. The flow-chart is a method embodiment for obtaining a plurality of samples from a plurality of subjects, the method comprising:
S11 -discarding the holding part according the first aspect of the invention, from the base part when a sample has been deposited in the associated sample vial,
S12 -initiating a disinfection process of the base part,
S13 -initiating a second sample obtaining process according the second aspect of the invention,
S14 -looping the following steps: S4, S6, S7, S8, S9, S10, S11, S12 and S13, according to Figure 7, completing n sample obtaining processes.

In a further embodiment, the associated sample obtaining element is inserted into an associated container, and while being inserted in said container, being disengaged from the holding part, leaving the sample obtaining element within said container.

In another, further embodiment, the associated sample obtaining element and the holding part may be assembled and bundled together prior to use of the invention so as to eliminate steps S6 and S8 according to Figure 7.

In another, further embodiment, the invention relates to a device for obtaining a sample from a subject. The device has a base part 10 with a base canal 13 extending into the base part 10, sensor means 12 being adapted to detect an element entering the base canal, a holding part 100 with a coupling end configured to interlock with the base part 10, a receiving end, and a holding canal 101 for holding an associated sample obtaining element 5. The device is arranged for holding an associated sample obtaining element 5, having a sample end 6 which protrudes from the receiving end of the holding part 100 when the associated sample obtaining element is inserted into the holding canal. The base part has fixation means configured to provide releasable fixation in an axial direction, to the associated sample obtaining element, the fixation means fixating the associated sample obtaining element with a force F. The fixation means has safety means configured to release fixation of the associated sample obtaining element 5, if a force Q exceeding F, is applied to the sample end of the associated sample obtaining element, and the device is adapted to provide a signal to the associated device if the safety means or the sensor means are activated by the associated sample obtaining element 5, due to an external force, provided to the sample end 6 of the associated sample element in a longitudinal or substantially longitudinal direction.

Although the present invention has been described in connection with the specified embodiments, it should not be construed as being in any way limited to the presented examples. The scope of the present invention is set out by the accompanying claim set. In the context of the claims, the terms "comprising" or "comprises" do not exclude other possible elements or steps. Also, the mentioning of references such as "a" or "an" etc. should not be construed as excluding a plurality. The use of reference signs in the claims with respect to elements indicated in the figures shall also not be construed as limiting the scope of the invention. Furthermore, individual features mentioned in different claims, may possibly be advantageously combined, and the mentioning of these features in different claims does not exclude that a combination of features is not possible and advantageous.

## Claims

1. A device for obtaining a sample from a subject, said device comprising:
- a base part (10) comprising a base canal (13) extending into the base part,
- sensor means (12) being adapted to detect an element entering the base canal,
- output means for outputting a signal from the sensor means to an associated device,
- a holding part (100), the holding part comprising a coupling end configured to interlocking with the base part, a receiving end, and a holding canal (101) for holding an associated sample obtaining element (5), wherein the base canal and the holding canal are arranged coaxially when the base part and the holding part are interlocked,
- an associated sample obtaining element, having a sample end (6) and a second end (7) opposite to the sample end and wherein the sample end protrudes from the receiving end of the holding part when the associated sample obtaining element is inserted into the holding canal,
- fixation means configured to provide releasable fixation in an axial direction, to the associated sample obtaining element, the fixation means fixating the associated sample obtaining element with a force F, the fixation means comprising safety means configured to release fixation of the associated sample obtaining element, if a force Q exceeding F, is applied to the sample end of the associated sample obtaining element, and
wherein the device is adapted to provide a signal to the associated device if the safety means or the sensor means are activated by the associated sample obtaining element, due to an external force, provided to the sample end of the associated sample element in a longitudinal or substantially longitudinal direction.

2. The device according to claim 1, in which the base part is configured to be mounted on an automatic positioning device, such as a robotic arm and wherein the output means are connected to a controller or data processing means of the automatic positioning device.

3. The device according to any of the preceding claims, in which the fixation means further comprise a resistive axial sliding mechanism, such as a spring mechanism for enabling an axial movement of the associated sample obtaining element, relative to the holding canal.

4. The device according to any of the preceding claims, in which the base part further comprise distance measuring means for measuring a distance D, the distance D being an axial movement between a start position and an end position of the associated sample obtaining element, relative to the holding canal, during a sample obtaining process.

5. The device according to any of the preceding claims, wherein the force F is a force of no more than 10 newton, preferably a force of no more than 3 newton, more preferably a force of no more than 1 newton and most preferably a force of no more than 0.8 newton.

6. The device according to any of the preceding claims, in which the holding part is manufactured from a disposable and/or recyclable material and/or biodegradable material.

7. The device according to any of the preceding claims, in which the base part is manufactured from, or coated with, a biocidal or antimicrobial material.

8. The device according to any of the preceding claims, in which the sample obtained is a mouth/throat swab.

9. The device according to any of the preceding claims, in which the safety means is a barrier or membrane, said barrier being penetrable by the force F applied by the second end of the associated sample obtaining element.

10. The device according to any of the preceding claims, in which the safety means is an electronic sensor, such as a strain gauge, the electronic sensor being configured to detect a force applied to the sample end and/or from the second end of the associated sample obtaining device in a longitudinal or substantially longitudinal direction.

11. The device according to any of the preceding claims, in which the fixation means and/ or the safety means is a magnetic device wherein the magnetic device is configured to detect a force applied to the sample end and/or from the second end of the associated sample obtaining device in a longitudinal or substantially longitudinal direction and upon detection of a force greater than F, release the fixation means and release an axial translation of the associated sample obtaining device.

12. A method for obtaining a sample from a subject, the method comprising:
- providing a base part comprising a base canal extending into the base part,
- providing sensor means being adapted to detect an element entering the base canal,
- providing output means for outputting a signal from the sensor means to an associated device,
- providing a holding part, the holding part comprising a coupling end configured to interlocking with the base part and a receiving end opposite to the coupling end, a holding canal for holding a sample obtaining element, wherein the base canal and the holding canal are arrange coaxially when the base part and the holding part are interlocked,
- providing fixation means configured to provide releasable fixation in an axial direction, to the associated sample obtaining element, the fixation means fixating the associated sample obtaining element with a force F, the fixation means comprising safety means configured to release fixation of the associated sample obtaining element, if a force Q exceeding F, is applied to the sample end of the associated sample obtaining element,
- providing the associated sample obtaining element, having a sample end and a second end opposite to the sample end and wherein the sample end protrudes from the receiving end of the holding part when the associated sample obtaining element is inserted into the holding canal and, by an external force,
- interlocking the holding part to the base part,
- inserting the associated sample obtaining element into the holding canal,
- obtaining a sample from the subject, such as from the subject's mouth or throat, such as a throat swab, and
wherein the method further comprises stopping the sample obtaining of the subject, if a force Q, which exceeds F, is applied to the sample end of the associated sample element in a longitudinal or substantially longitudinal direction and release fixation of the associated sample obtaining element.

13. The method according to claim 13, further comprising:
- providing automatic positioning means, such as a robotic arm, the position means being interlocked with the base part,
- inserting the associated sample obtaining element into an associated container, such as a sample vial.

14. The method according to any of claims 13 to 14 further comprising:
- moving the device according to any of the preceding claims in at least a first direction transverse to a longitudinal axis of the associated container and breaking or snapping at least the sample end of the associated sample obtaining element into the associated container.

15. The method according to any of claims 13 to 15, in which a plurality of samples are to be obtained from one or more subjects, the method further comprising:
- discarding the holding part according to any of claims 1 to 12 from the base part when a sample has been deposited in the associated sample vial,
- initiating a disinfection process of the base part,
- initiating a second sample obtaining process according to any of claims 13 to 15,
- looping the method according to any of claims 13 to 15, completing *n* sample obtaining processes.

16. A kit for obtaining a sample from a subject, the kit comprising:
- a device according to any of claims 1 to 12,
- a sample obtaining element comprising a sample end and a second end, the sample obtaining element being configured to break/snap at a breaking/snapping section of the sample obtaining element, if shear force exceeding between 0.1 and 20 newton, preferably between 0.5 and 10 newton or more preferably between 0.5 and 4 newton is applied to the sample obtaining element,
wherein the breaking/snapping section of the sample obtaining element is positioned at the receiving end of the holding part, when the sample obtaining element is inserted into the holding part, pressing against the safety means.
